# EUROPEAN PATENT SPECIFICATION

(11) **EP 4 042 983 B1**
(45) Date of publication and mention of the grant of the patent: **04.09.2024**
(21) Application number: 20888430.4
(22) Date of filing: 14.10.2020
(51) Int. Cl.: A61F 2/95, A61B 17/04

(54) **OPERATING SYSTEM AND IMPLANT DELIVERY SYSTEM**
OPERATIONSSYSTEM UND IMPLANTATABGABESYSTEM
SYSTÈME D'EXPLOITATION ET SYSTÈME DE POSE D'IMPLANT

(30) Priority: 13.11.2019 CN 201911103520
(43) Date of publication of application: 17.08.2022
(73) Proprietor: MicroPort Urocare (Jiaxing) Co., Ltd., Nanhu District Jiaxing Zhejiang 314006 (CN)
(72) Inventor: WANG, Zhen, Jiaxing, Zhejiang 314006 (CN); FU, Zhenzhong, Jiaxing, Zhejiang 314006 (CN); FU, Jiawei, Jiaxing, Zhejiang 314006 (CN); GENG, Ran, Jiaxing, Zhejiang 314006 (CN); QUE, Yiyun, Jiaxing, Zhejiang 314006 (CN); ZHANG, Yi, Jiaxing, Zhejiang 314006 (CN)
(74) Representative: Marks & Clerk LLP
(86) International application number: PCT/CN2020/120938
(87) International publication number: WO 2021/093506

(56) References cited:
- CN-A- 101 795 641
- CN-A- 102 112 064
- CN-A- 109 833 126
- CN-A- 110 179 571
- CN-A- 110 267 627
- CN-A- 110 584 853
- US-A1- 2011 060 349
- US-A1- 2011 196 388
- US-A1- 2012 215 171
- US-A1- 2018 344 501

## Description

### TECHNICAL FIELD

The present invention relates to the field of medical devices and, more particularly, to an operating system for use in an implant delivery system and an implant delivery system.

### BACKGROUND

Benign prostatic hyperplasia (BPH) is a common condition affecting middle- and older-aged men. Major manifestations of BPH include prostatic stromal and glandular hyperplasia, benign prostatic enlargement (BPE), lower urinary tract symptoms (LUTS), bladder outlet obstruction (BOO), etc.

Currently, BPH is treated by medication or surgery. Available surgical therapies include 1) transurethral resection, 2) suprapubic or retropubic resection and 3) laser enucleation or resection of the prostate and 4) minimally invasive implants, among others. A conventional permanently implantable product for BPH treatment, when implanted into the body using a delivery system, can mechanically hold apart the prostatic lobes and open up the obstructed urethra. The in vivo implantation approach can provide continuous symptom relief to BPH patients and dispenses with resection or ablation of prostatic tissue. Therefore, it can alleviate patients' pain and return them to normal life as soon as possible.

The conventional delivery system utilizes a puncture needle to deliver the implant into the human body, and the puncture needle is then withdrawn from the body. Finally, a cutting and pushing mechanism in the delivery system cuts excess tether from the implant. However, due to a deficient design, a physician tends to mistakenly operate the delivery system during use. Consequently, the cutting and pushing mechanism may cut the tether at the same time as the withdrawal of the puncture needle, leading to failure of implantation. Moreover, during use of the conventional delivery system, the implant tends to be stuck and fails to be released due to device faults. All these problems affect reliability of the delivery system during use.

Document US2011060349 A1 discloses a tool that includes a case assembly enclosing an anchor delivery and assembly structure, a needle spool assembly and a suture spool assembly. Extending from the case assembly is a shaft assembly. Also, extending through the shaft assembly are a pusher assembly, a needle, and a cutter assembly. Operatively associated with the needle spool and suture spool assemblies are a needle actuator and a needle retraction actuator (e.g., a lever). An assembly actuator is operatively associated with the anchor assembly structure. Safety lock and lock-out structures are also operatively associated with the needle actuator and assembly actuator. Activation of the needle actuator accomplishes the advancement of a needle assembly and a first component of an anchor assembly attached to a connector member, to an interventional site. Activation of the needle retraction actuator withdraws the needle assembly leaving the first component of the anchor assembly at the interventional site. Thereafter, manipulation of the assembly actuator results in lockingly engaging a second anchor component with the connector member and cutting the connector member below the second anchor component.

### SUMMARY OF THE INVENTION

It is an objective of the present invention to provide an operating system for use in an implant delivery system and an implant delivery system which ensure that, during the release of an implant, a tether is cut strictly after a puncture needle has been withdrawn, thus avoiding failed release due to mistaken operation, ensuring safety of use of the device, and reducing patient damage caused by device faults and the associated risk. Moreover, the operating system overcomes the problem of failed release of a stuck implant and imparts higher reliability to the device during its use.

To this end, the operating system provided in the present invention, as defined in claim 1, is for use in an implant delivery system and comprises a housing and a trigger. The housing defines an internal cavity in which part of the trigger is movably disposed. The trigger includes a withdrawal lock, a cutting lock and a cutting and pushing mechanism. The cutting and pushing mechanism is configured to cut off a connecting element from an implant delivered by the implant delivery system.

The operating system has an initial configuration.

The trigger configured so that, in the initial configuration, the withdrawal lock is locked and locks the cutting lock which locks the cutting and pushing mechanism, and that when the withdrawal lock is unlocked, the withdrawal lock releases the locked cutting lock under the action of an external force, followed by the cutting lock releasing the locked cutting and pushing mechanism under the action of an external force.

The trigger further includes a limiting mechanism, a release member and a transmission member, the release member rotatably disposed in the internal cavity, the transmission member movably disposed in the internal cavity and coupled to the withdrawal lock,
wherein the limiting mechanism is configured to lock the withdrawal lock in the initial configuration, and the release member is configured to, when rotated in a first direction over a predetermined angle under the action of an external force, urge the limiting mechanism to deform, thereby releasing the locked withdrawal lock, followed by the withdrawal lock releasing the locked cutting lock under the action of an external force and simultaneously driving movement of the transmission member, which causes the release member to rotate in a second direction opposite to the first direction.

Optionally, the trigger may further include a release lock configured to lock the release member in the initial configuration.

Optionally, the release lock may include a first manipulation mechanism and a second manipulation mechanism, the first manipulation mechanism is configured to lock the second manipulation mechanism, which is configured to lock the release member, in the initial configuration.

Optionally, the housing may be provided therein with a first window in communication with the internal cavity, wherein the operating system further includes a manual manipulation mechanism, and
wherein the trigger is further configured so that the second manipulation mechanism releases the locked release member under the action of a force exerted on the second manipulation mechanism by the manual manipulation mechanism inserted into the internal cavity from the first window.

Optionally, the second manipulation mechanism may include a manipulation member and a first limiting element, the manipulation member disposed partially outside the housing and partially in the internal cavity, the first limiting element disposed in the internal cavity,
wherein the second manipulation mechanism is configured so that, in the initial configuration, the manipulation member abuts against the first manipulation mechanism, with the first limiting element abutting against both the manipulation member and the release member, thereby locking the release member, and that under the action of an external force, the manipulation member drives the first limiting element to move away from the release member, thereby releasing the locked release member.

Optionally, the first window in the housing may be arranged in positional correspondence with the manipulation member,

wherein the second manipulation mechanism is further configured so that the manipulation member is able to drive the first limiting element to move away from the release member and thus release the locked release member under the action of a force exerted on the manipulation member by the manual manipulation mechanism inserted into the internal cavity from the first window.

Optionally, the first window in the housing may be arranged in positional correspondence with the first limiting element,
wherein the second manipulation mechanism is further configured so that the first limiting element is able to release the locked release member under the action of a force exerted on the first limiting element by the manual manipulation mechanism inserted into the internal cavity from the first window.

Optionally, the first limiting element may be made of an elastic material.

Optionally, the housing may be provided therein with a second window in communication with the internal cavity, wherein the operating system further includes a manual manipulation mechanism, and
wherein the trigger is further configured so that the limiting mechanism is able to deform to release the locked withdrawal lock under the action of a force exerted on the limiting mechanism by the manual manipulation mechanism inserted into the internal cavity from the second window.

Optionally, the limiting mechanism may include a first limiting post and a second limiting post,
wherein the limiting mechanism is configured so that the first limiting post limits movement of the withdrawal lock in a third direction in the initial configuration and deforms to release the limited withdrawal lock under the action of an external force and that the second limiting post limits movement of the withdrawal lock in a fourth direction opposite to the third direction.

Optionally, the second window in the housing may be arranged in positional correspondence with the first limiting post,
wherein the first limiting post is able to deform under the action of a force exerted thereon by the manual manipulation mechanism inserted into the internal cavity from the second window.

Optionally, the first limiting post may be configured to abut against the transmission member and thereby limit movement of the withdrawal lock in the third direction.

Optionally, the cutting and pushing mechanism may include a slide channel, a first push block, a biased push element, a second push block and a second limiting element. The slide channel disposed in the internal cavity, the first and second push blocks movably provided on the slide channel, the biased push element coupled to both the first and second push blocks and configured to store potential energy, the second limiting element rotatably disposed in the internal cavity,
wherein the cutting and pushing mechanism is configured so that: in the initial configuration, the first push block is coupled to the cutting lock and is thus locked, the second limiting element is disposed between the first push block and the second push block, and the second limiting element abuts against the second push block to lock the second push block, and under the action of an external force exerted on the cutting lock, the first push block is disengaged from the cutting lock and urged by the biased push element to move within the slide channel toward the second push block until it comes into contact with and exerts a force on the second limiting element, which causes the second limiting element to move away from the second push block and thus releases the locked second push block, followed by the second push block being urged by the biased push element to move toward the first push block.

Optionally, the housing may be provided therein with a third window in communication with the internal cavity, wherein the operating system further includes a manual manipulation mechanism, and
wherein the cutting and pushing mechanism is further configured so that the second limiting element is able to move away from the second push block under the action of a force exerted on the second limiting element by the manual manipulation mechanism inserted into the internal cavity from the third window.

Optionally, the withdrawal lock may be further configured to lock the second limiting element in the initial configuration and release the locked second limiting element under the action of an external force.

Optionally, the release member may define a first abutment feature adapted to deform the limiting mechanism by exerting a force thereon.

Optionally, the transmission member may be rotatably disposed in the housing so as to be coaxial with the release member and define a second abutment feature adapted to be brought into contact with the release member and drive it to rotate.

Optionally, the release member may further define a third abutment feature adapted to be brought into contact with the second abutment feature of the transmission member to enable the transmission member to drive the release member to rotate.

Optionally, the transmission member may define a protrusion adapted to be brought into contact with the limiting mechanism and thus limit movement of the withdrawal lock in a third direction.

Optionally, the transmission member may be provided with ratchet teeth and the housing with a contact tab, which is disposed in the internal cavity and brought into contact with the ratchet teeth.

Optionally, the withdrawal lock may be rotatably disposed on the housing.

Additionally or alternatively, the cutting lock may be rotatably disposed on the housing.

Additionally or alternatively, the first manipulation mechanism may be rotatably disposed on the withdrawal lock.

Additionally or alternatively, the second manipulation mechanism may be rotatably disposed on the withdrawal lock.

Optionally, the withdrawal lock may be provided with a push slot which is located outside the housing, wherein the first manipulation mechanism is provided with a push stud which is movably disposed in the push slot.

Optionally, the housing may includes a first housing and a second housing, which are joined together to delimit the internal cavity therebetween.

To the above end, the implant delivery system provided in the present invention is for delivering an implant provided with a connecting element and includes a puncture needle, a delivery tube and the operating system as defined above.

The delivery tube defines a first lumen and a second lumen. A first portion of the implant is received in the puncture needle, and the puncture needle is movably disposed in the first lumen. A second portion of the implant is disposed in the second lumen. Part of the cutting and pushing mechanism of the operating system is movably disposed in the second lumen. The cutting and pushing mechanism is configured to cut off the connecting element from the implant.

To the above end, the implant delivery method provided in the present disclosure is based on the above implant delivery system, which is in the initial configuration before the implant is delivered and includes:
releasing the puncture needle;
releasing the locked withdrawal lock, releasing the locked cutting lock by exerting an external force on the withdrawal lock, and withdrawing the puncture needle; and
releasing the cutting and pushing mechanism by exerting an external force on the cutting lock so that the cutting and pushing mechanism is enabled to cut off the connecting element from the implant, thus completing the delivery of the implant.

Optionally, the trigger of the operating system may further include a release member, a transmission member and a limiting mechanism, the release member coupled to the puncture needle, the transmission member coupled to the withdrawal lock, the limiting mechanism configured to lock the withdrawal lock in the initial configuration, wherein:
the puncture needle is released by exerting an external force on the release member to drive it to rotate in a first direction;
when the release member is rotated over a predetermined angle, the release member hits the limiting mechanism, so as to deform the limiting mechanism and release the locked withdrawal lock; and
at the same time as an external force is applied to the withdrawal lock to unlock the cutting lock from the withdrawal lock, the withdrawal lock drives movement of the transmission member, which causes the release member to rotate in a second direction opposite to the first direction to withdraw the puncture needle.

Optionally, the trigger may further include a release lock which is adapted to lock the release member in the initial configuration,
wherein prior to the application of an external force to the release member, the implant delivery method further includes unlocking the release member from the release lock by exerting an external force on the release lock.

Optionally, the release lock may include a first manipulation mechanism and a second manipulation mechanism, the first manipulation mechanism is configured to lock the second manipulation mechanism, which is configured to lock the release member, in the initial configuration,
wherein unlocking the release member from the release lock by exerting an external force on the release lock includes: unlocking the second manipulation mechanism from the first manipulation mechanism by exerting an external force on the first manipulation mechanism; and then unlocking the release member from the second manipulation mechanism by exerting an external force on the second manipulation mechanism.

Optionally, the housing may be provided therein with a first window in communication with the internal cavity, wherein the operating system further includes a manual manipulation mechanism; and
wherein in the event of the implant being stuck in the course of unlocking the release member from the second manipulation mechanism, the manual manipulation mechanism is inserted into the internal cavity from the first window to exert a force on the second manipulation mechanism and thereby release the locked release member.

Optionally, the housing may be provided therein with a second window in communication with the internal cavity, wherein: the operating system further includes a manual manipulation mechanism; and
in the event of the implant being stuck in the course of releasing the locked withdrawal lock as a result of the release member hitting the limiting mechanism, the manual manipulation mechanism is inserted into the internal cavity from the second window to exert a force on the limiting mechanism, which deforms the limiting mechanism and thus releases the locked withdrawal lock.

Optionally, the housing may be provided therein with a third window in communication with the internal cavity, wherein: the operating system further includes a manual manipulation mechanism; and
in the event of the implant being stuck in the course of unlocking the cutting and pushing mechanism from the cutting lock, the manual manipulation mechanism is inserted into the internal cavity from the third window to release the locked cutting and pushing mechanism by exerting a force thereon.

Compared to the prior art, the operating system and the implant delivery system of the present invention have the following advantages:
First, the implant delivery system includes the operating system and a delivery tube coupled to the operating system, and the operating system includes a housing and a trigger. The housing defines an internal cavity, and the trigger includes a withdrawal lock, a cutting lock and a cutting and pushing mechanism. The operating system has an initial configuration, in which the withdrawal lock is locked and locks the cutting lock which in turn locks the cutting and pushing mechanism. The delivery tube is configured to receive therein a puncture needle and an implant. In order to deliver the implant by the implant delivery system, the puncture needle is first released to position the implant on an object, and the user then releases the locked withdrawal lock to withdraw the puncture needle. At the same time, the unlocked withdrawal lock releases the locked cutting lock, and an external force is then exerted on the cutting lock to release the cutting and pushing mechanism from the cutting lock. As a result, the cutting and pushing mechanism is enabled to cut off the connecting element from the implant, thus completing the delivery of the implant. In the implant delivery process performed by the delivery system of the present invention, the unlock and triggering actions are taken in a predetermined order, avoiding failed release due to a wrong order and improving the reliability and safety of the implant delivery system.
Second, first, second and third windows may be provided in the housing, and the operating system may further include a manual manipulation mechanism. The manual manipulation mechanism can be inserted into the internal cavity from the first window to manually release the locked release member. It can also be inserted into the internal cavity from the second window to manually release the locked withdrawal lock. It can further be inserted into the internal cavity from the third window to manually release the locked cutting and pushing mechanism. In this way, the implant can be avoided from being stuck during any of the triggering actions and thus from failing to be released. This makes the implant delivery system even more reliable.

### BRIEF DESCRIPTION OF THE DRAWINGS

Fig. 1 schematically illustrates how an implant according to an embodiment of the present invention is deployed in the prostate;
Fig. 2 is a schematic diagram illustrating the structure of a leading portion of a puncture needle in an implant delivery system according to an embodiment of the present invention;
Fig. 3 is an enlarged partial schematic view of the puncture needle of Fig. 2;
Fig. 4 is a schematic diagram illustrating the structure of the implant delivery system, as viewed along a predetermined direction, according to an embodiment of the present invention;
Fig. 5 is a schematic diagram illustrating the structure of the implant delivery system of Fig. 4, as viewed along another predetermined direction;
Fig. 6 is a schematic diagram illustrating a delivery tube in the implant delivery system of Fig. 4;
Fig. 7 shows a cross-sectional view of the delivery tube of Fig. 6 taken along A-A;
Fig. 8 is a schematic diagram illustrating the structure of an operating system according to an embodiment of the present invention, in which a cutting and pushing mechanism is not completely shown;
Fig. 9 is a schematic diagram illustrating the structure of a first manipulation mechanism in the operating system according to an embodiment of the present invention;
Fig. 10 is a schematic diagram illustrating the structure of a manipulation member of a second manipulation mechanism in the operating system according to an embodiment of the present invention;
Fig. 11 is a schematic diagram illustrating the structure of a withdrawal lock in the operating system according to an embodiment of the present invention;
Fig. 12 is a schematic diagram illustrating the structure of a cutting lock in the operating system according to an embodiment of the present invention;
Fig. 13 is a schematic diagram illustrating the structure of a release member in the operating system according to an embodiment of the present invention;
Fig. 14 is a schematic diagram illustrating the structure of a transmission member in the operating system according to an embodiment of the present invention;
Fig. 15 is a schematic illustration of the operating system in a configuration in which the puncture needle is being released according to an embodiment of the present invention, in which the cutting and pushing mechanism is not completely shown and the transmission member is not shown;
Fig. 16 is a schematic illustration of the operating system in a configuration in which the transmission member is driving the release member to rotate in an opposite direction according to an embodiment of the present invention, in which the cutting and pushing mechanism is not completely shown and a first limiting element in the release member is not shown;
Fig. 17 is a schematic diagram illustrating the structure of the delivery system, with the puncture needle having been released, according to an embodiment of the present invention;
Fig. 18 is a schematic diagram illustrating the structure of the cutting and pushing mechanism in the operating system, with a first push block having hit a second limiting element and thus caused it to move away from the second push block, according to an embodiment of the present invention, in which a slide channel is not shown;
Fig. 19 is a schematic illustration of the cutting and pushing mechanism in the implant delivery system, which is being accommodated in a second lumen, according to an embodiment of the present invention;
Fig. 20 is a partial schematic view of a housing in the operating system according to an embodiment of the present invention, in which a first window and a second window are shown;
Fig. 21 is a partial schematic view of the housing in the operating system according to an embodiment of the present invention, in which a third window is shown;
Fig. 22 schematically illustrates a manual manipulation mechanism in the operating system according to an embodiment of the present invention;
Fig. 23 is a schematic diagram illustrating the structure of a tube fitting in the implant delivery system according to an embodiment of the present invention;
Fig. 24 is a schematic diagram illustrating the structure of a sheath lock in the implant delivery system according to an embodiment of the present invention; and
Fig. 25 is a schematic diagram illustrating the structure of an endoscope lock in the implant delivery system according to an embodiment of the present invention.

### DETAILED DESCRIPTION

Objects, features and advantages of the present invention will become more apparent upon reading the following more detailed description thereof with reference to the accompanying drawings. Note that the figures are provided in a very simplified form not necessarily drawn to exact scale for the only purpose of facilitating easy and clear description of the disclosed embodiments.

As used herein, the singular forms "a", "an" and "the" include plural referents and the term "plurality" means two or more, unless the context clearly dictates otherwise. As used herein, the term "or" is generally employed in the sense of "and/or", unless the context clearly dictates otherwise. The terms "installation", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two components. Those of ordinary skill in the art can understand the specific meanings of the above-mentioned terms herein, depending on their context. Like numerals indicate like elements throughout the accompanying drawings.

Embodiments of the present invention provide an implant delivery system and an operating system for use in the implant delivery system. The implant delivery system is adapted to deliver a medical implant into the human body. For ease of understanding, the implant delivery system is described below in the exemplary context of its use in the treatment of benign prostatic enlargement.

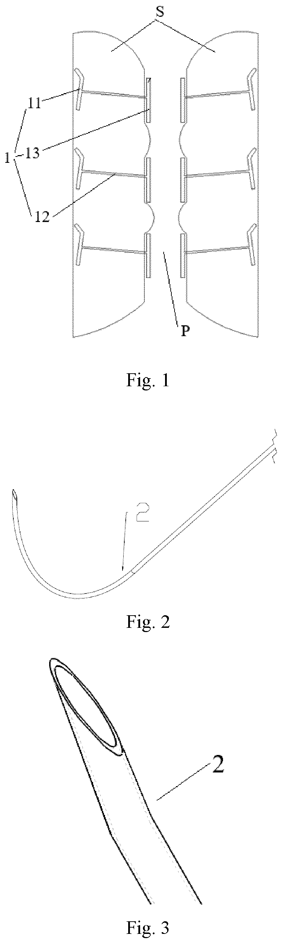

In one embodiment, the implant delivery system is used to implant a medical implant into prostatic tissue so that the implant mechanically holds apart the prostatic lobes and opens up the obstructed urethra. Fig. 1 schematically illustrates the medical implant that has been implanted in the prostate. As shown in Fig. 1, the implant delivery system includes the implant 1 including, sequentially coupled together, a distal anchoring element 11, a connecting element 12 and a proximal anchoring element 13. The distal anchoring element 11 may be securely assembled with the connecting element 12 by crimping and pre-loaded in a puncture needle (not shown in Fig. 1), and the proximal anchoring element 13 may be pre-loaded in a delivery tube (not shown in Fig. 1). In order to deliver the implant 1 using the implant delivery system, the puncture needle carrying the distal anchoring element 11 is caused to penetrate into the prostate S through the capsule thereof and position the distal anchoring element 11 at a distal location of the prostate S. The implant delivery system is then operated to withdraw the puncture needle and tension the distal anchoring element 11. After that, it is further operated to advance the proximal anchoring element 13 into engagement with the connecting element 12 so that the proximal anchoring element 13 is positioned at a proximal location of the prostate S. Finally, the implant delivery system is operated to cut off an unwanted portion of the connecting element 12 from the implant 1, thus completing the delivery process. Depending on the patient's conditions, 4-6 implants 1 may need to be deployed on each of the prostatic lobes. The deployed implants 1 hold apart the lobes and open up the obstructed urethra P. Here, the term "proximal" refers to a prostate location adjacent to the urethra, and in contrast to "proximal", the term "distal" refers to a prostate location more distant from the urethra.

In order to implant the distal anchoring element 11 into prostatic tissue, the puncture needle is generally inserted into prostatic tissue to a depth of 30-35 mm. As shown in Fig. 2, in order to facilitate the insertion of the puncture needle 2 into prostatic tissue, a leading portion of the puncture needle 2 is shaped like an arc preferably of 90°. Additionally, as shown in Fig. 3, the leading portion of the puncture needle 2 defines a sharp beveled tip with a bevel angle, which may be 15- 20°, for example, 17°, 18°, 19° or the like. The leading portion is a portion of the puncture needle 2 that is first brought into contact with prostatic tissue.

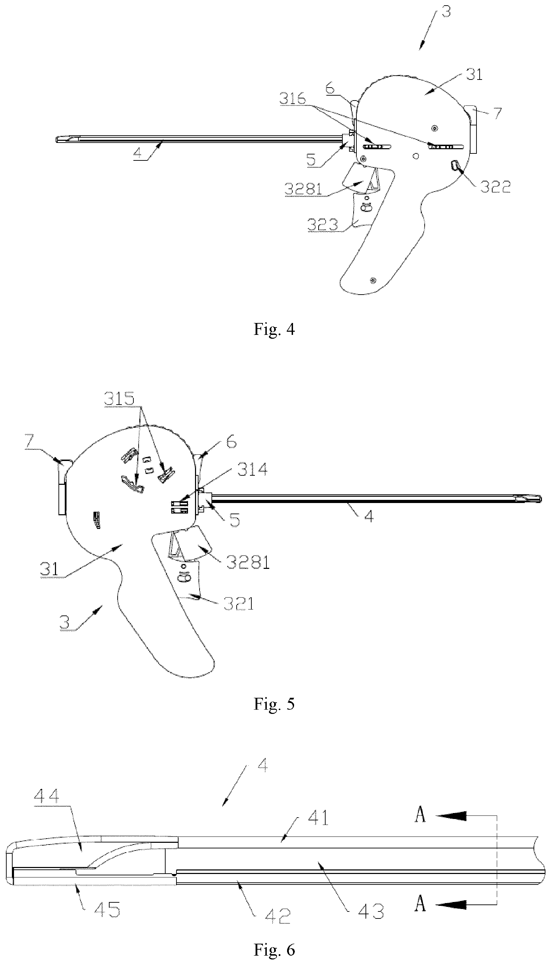

Reference is now made to Figs. 4 to 8. In one embodiment of the present invention, the implant may be released manually. Specifically, the implant delivery system includes an operating system 3 and a delivery tube 4, which are coupled together. The delivery tube 4 defines at least a first lumen 41 and a second lumen 42. The first lumen 41 is adapted to receive the puncture needle (not shown in Figs. 3 and 4) in such a manner that there is a clearance between the puncture needle and a wall of the lumen, which allows the puncture needle to move axially in the first lumen 41. The second lumen 42 is adapted to receive the proximal anchoring element (not shown in Figs. 3 and 4) while allowing it to move axially in the second lumen 42. The operating system 3 includes a housing 31 and a trigger 32. The housing 31 has an internal cavity (not labeled in the figures). Some features of the trigger 32 are arranged in the internal cavity, while the remaining features are arranged outside the housing 31. This arrangement of features of the trigger32 may vary depending on the needs of particular applications. The delivery tube 4 is coupled to the housing 31 and brought into communication with the internal cavity. The puncture needle is coupled to the trigger 32, and the trigger 32 is adapted to release and withdraw the puncture needle and to cut off an undesired portion of the connecting element 12 from the implant. The positioning of the trigger 32 relative to, and its method of coupling to, the puncture needle and the proximal anchoring element, as well as how the trigger 32 operates, will be detailed below.

Optionally, in the present embodiment, the housing 31 is composed of a first housing (not labeled in the figures) and a second housing (not labeled in the figures), which are joined together to delimit the internal cavity therebetween. This multi-piece structure allows easy assembly of the operating system 3 and simplifies its fabrication.

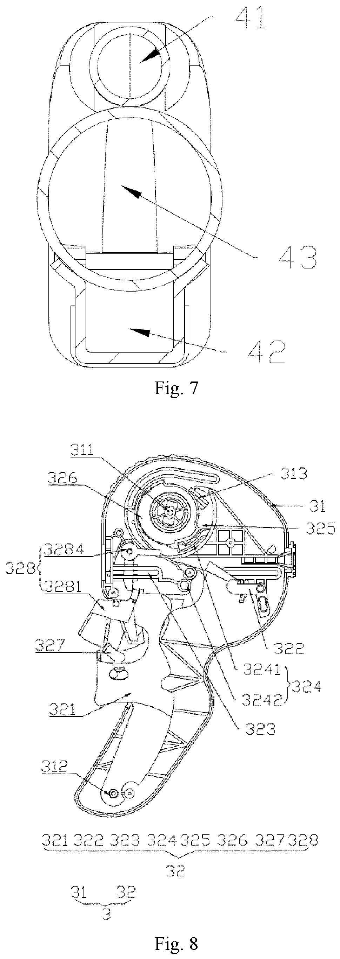

As shown in Fig. 8, the trigger 32 includes at least a withdrawal lock 321, a cutting lock 322 and a cutting and pushing mechanism 323. Both the withdrawal lock 321 and the cutting lock 322 are partially disposed in the internal cavity, and the cutting and pushing mechanism 323 is adapted to cut off an undesired portion of the connecting element 12 from the implant. Before the implant is released, the operating system 3 is in an initial configuration in which the withdrawal lock 321 is in a locked position where it locks the cutting lock 322 and the locked cutting lock 322 in turn locks the cutting and pushing mechanism 323.When the withdrawal lock 321 is unlocked, the withdrawal lock 321 can release the locked cutting lock 322 under the action of an external force. As a result, the cutting lock 322 can release the locked cutting and pushing mechanism 323 under the action of an external force. It will be appreciated that when the operating system 3 is used in the implant delivery system to deliver an implant, the withdrawal lock 321 is unlocked after the puncture needle is released. That is, in the present embodiment, in the initial configuration, the withdrawal lock 321 locks the cutting lock 322 without undesirable unlocking of the cutting lock 322 prior to or during the release of the puncture needle. Here, the term "external force" refers to a force exerted on the concerned element by another element or mechanism.

The trigger 32 further includes a limiting mechanism 324, a release member 325 and a transmission member 326. The release member 325 is rotatably disposed in the internal cavity and coupled to the puncture needle. The transmission member 326 is movably disposed in the internal cavity and coupled to the withdrawal lock 321. In the initial configuration, the limiting mechanism 324 locks the withdrawal lock 321. When the release member 325 is rotated in a first direction over a predetermined angle by an external force, the puncture needle is released to penetrate into prostatic tissue. Upon the release member 325 being rotated over the predetermined angle, it hits the limiting mechanism 324 and urges it to deform, thus releasing the locked withdrawal lock 321. As a result, the withdrawal lock 321 can release the locked cutting lock 322 under the action of an external force. At the same time, it drives the transmission member 326 to move, which in turn causes the release member 325 to move in a second direction that is opposite to the first direction. Thus, after the release member 325 releases the locked withdrawal lock 321 from the limiting mechanism 324, the withdrawal lock 321 releases the locked cutting lock 322 and simultaneously causes movement of the transmission member 326, which in turn causes the release member 325 to rotate in an opposite direction to withdraw the puncture needle. Finally, the cutting lock 322 releases the locked cutting and pushing mechanism 323 to allow it to perform its intended function. It will be appreciated that, in the present embodiment, if the first direction is clockwise, the second direction will be counterclockwise; and vice versa.

In other words, when the operating system 3 of the present embodiment is used in the implant delivery system, before the implant is released, the trigger 32 is in the initial configuration where the limiting mechanism 324, the withdrawal lock 321, the cutting lock 322 and the cutting and pushing mechanism 323 are interlocked together. Only after the release member 325 is rotated by an external force to release the puncture needle, will the withdrawal lock 321 and then the cutting lock 322 be unlocked. In this way, those components in the delivery system are released completely in a predetermined order. This interlocking design has the advantage that those components are unlocked and triggered according to a predetermined order during the implant delivery process of the delivery system, avoiding failed release due to a wrong unlocking order and improving the reliability and safety of the implant delivery system.

Optionally, the trigger 32 further includes a release lock adapted to lock the release member 325 in the aforementioned initial configuration. Specifically, the release lock includes a first manipulation mechanism 327 and a second manipulation mechanism 328. In the initial configuration, the first manipulation mechanism 327 locks the second manipulation mechanism 328, which in turn locks the release member 325.

In practice, the first manipulation mechanism 327 may be moved by an external force to unlock the second manipulation mechanism 328, and the second manipulation mechanism 328 may be moved by an external force to unlock the release member 325. The withdrawal lock 321 may be moved by an external force to unlock the cutting lock 322. The cutting lock 322 may be moved by an external force to unlock the cutting and pushing mechanism 323.

Optional structures of the various components in the trigger 32 and how they are assembled together will be described below with reference to Figs. 8 to 15. It should be understood that the specific structures of the various components in the trigger 32 and their assembly method described below are merely some but not all of the possible implementations of the present invention and thereby should not be construed as limiting the invention in any sense.

Generally, the withdrawal lock 321 is rotatably disposed on the housing 31, the first manipulation mechanism 327 is rotatably disposed on the withdrawal lock 321, the second manipulation mechanism 328 is rotatably disposed on the withdrawal lock 321 and the cutting lock 322 is rotatably disposed on the housing 31.

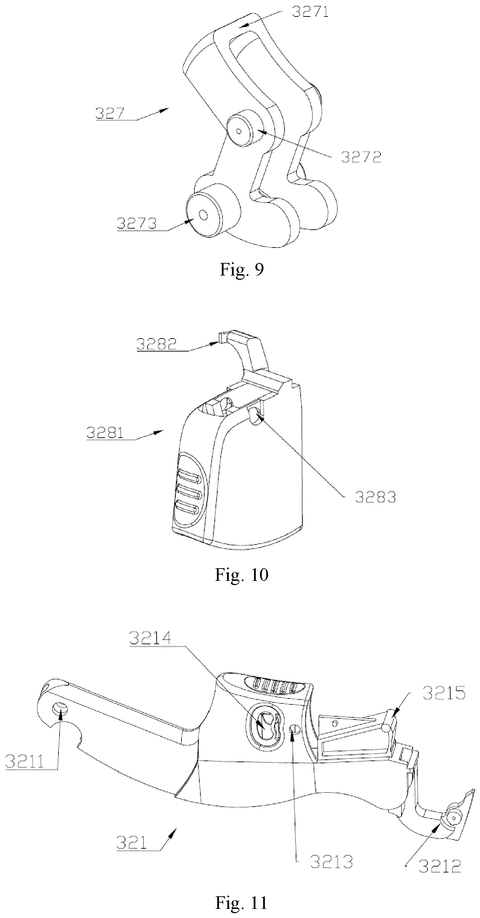

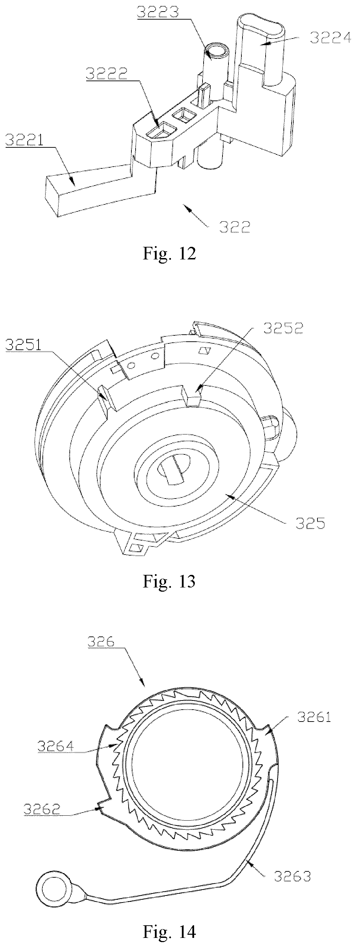

Specifically, referring to Fig. 9, the first manipulation mechanism 327 defines a third limiting element 3271 and is provided thereon with a first circular rotary disc 3272 and a first push member 3273. Referring to Fig. 10, the second manipulation mechanism 328 may include a manipulation member 3281 defining a fourth limiting element 3282 and a first circular accommodating recess 3283. Referring to Fig. 11, the withdrawal lock 321 has an elongate structure with a first end and an opposing second end. The first end defines a second circular accommodating recess 3211, and the second end defines a fifth limiting element 3212. The fifth limiting element 3212 may be a curved finger. A middle section of the withdrawal lock 321 defines a third circular accommodating recess 3213, a push slot 3214 and a second circular rotary disc 3215. Referring to Fig. 12, the cutting lock 322 defines a sixth limiting element 3221, a locking hook 3222, a third circular rotary disc 3223 and a second push member 3224.

Referring to Fig. 13, the release member 325 is essentially in the shape of a round disk and defines a first abutment feature 3251 and a third abutment feature 3252. Referring to Fig. 14, the transmission member 326 defines a second abutment feature 3261, a protrusion 3262 and a tether 3263.

With continued reference to Fig. 8, the release member 325 is disposed over a central shaft 311 in the internal cavity so that it can rotate about the central shaft 311 under the action of an external force. The transmission member 326 is also disposed over the central shaft 311 so as to be able to rotate about the central shaft 311 under the action of an external force. It should be understood that, in the present embodiment, the release member 325 and the transmission member 326 may be assembled in the internal cavity in a similar manner as conventional implant delivery systems.

With continued reference to Fig. 8, in conjunction with Figs. 11 and 14, a fourth circular rotary disc 312 and a fourth circular accommodating recess (not shown in the figures) are further disposed in the internal cavity. The second circular accommodating recess 3211 engages the fourth circular rotary disc 312, and the fifth limiting element 3212 is coupled to the tether 3263. Referring to Figs. 9 and 11, the first circular rotary disc 3272 engages the third circular accommodating recess 3213, and the first push member 3273 is movably disposed in the push slot 3214. Referring to Figs. 10 and 11, the first circular accommodating recess 3283 engages the second circular rotary disc 3215. Referring to Fig. 12, the third circular rotary disc 3223 engages the fourth circular accommodating recess, and the second push member 3224 protrudes out of the housing 31.

With continued reference to Fig. 8, more specifically, the second manipulation mechanism 328 may include a first limiting element 3284, which is rotatably disposed in the internal cavity and located between the release member 325 and the manipulation member 3281. The limiting mechanism 324 includes a first limiting post 3241 and a second limiting post 3242. The first limiting post 3241 may be formed of an elastic material.

Referring to Figs. 8 and 9, in the initial configuration, the third limiting element 3271 abuts against the manipulation member 3281, thereby locking the second manipulation mechanism 328. Referring to Figs. 8 and 10, the fourth limiting element 3282 abuts against the first limiting element 3284 which in turn abuts against the release member 325, thereby locking the release member 325. Referring to Figs. 8 and 14, the first limiting post 3241 can limit movement of the withdrawal lock 321 in a third direction. For example, the first limiting post 3241 may accomplish this by abutting against the protrusion 3262 in the transmission member 326. Referring to Figs. 8 and 11, the second limiting post 3242 may abut against the withdrawal lock 321. For example, the second limiting post 3242 may abut against the fifth limiting element 3212, thereby limiting movement of the withdrawal lock 321 in a fourth direction. In this way, the withdrawal lock 321 is locked by the limiting mechanism 324. Referring to Figs. 11 and 12, the fifth limiting element 3212 abuts against the cutting lock 322. Preferably, the fifth limiting element 3212 abuts against the sixth limiting element 3221, thereby locking the cutting lock 322. This arrangement not only allows a simpler structure of the trigger 32 and a reduced size of the operating system 3, but also enables unlocking of the cutting lock 322 simply as a result of separating the fifth limiting element 3212 from the sixth limiting element 3221 in accordance with the present embodiment. Compared to other unlocking approaches such as structural breakage or structural deformation, the above various locked mechanisms can be more easily unlocked, reducing the risk of the implant being stuck during its release. In addition, referring to Fig. 13, in the initial configuration, there is a circumferential clearance left between the first abutment feature 3251 and the second limiting post 3242 and another circumferential clearance left between the third abutment feature 3252 and the second abutment feature 3261. Further, in the present embodiment, the third and fourth directions are opposite to each other. For example, the third direction may be clockwise and the fourth direction may be counterclockwise; and vice versa.

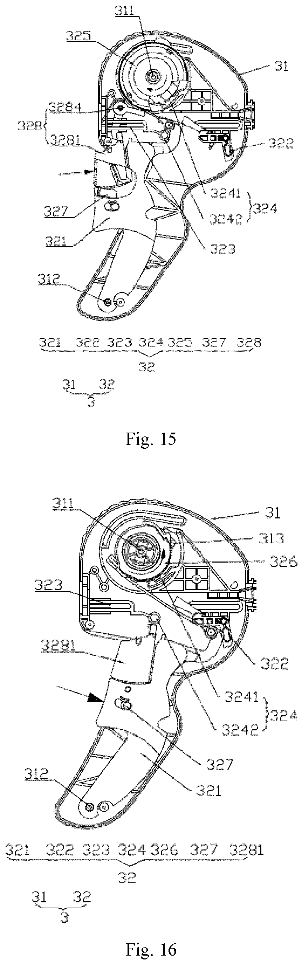

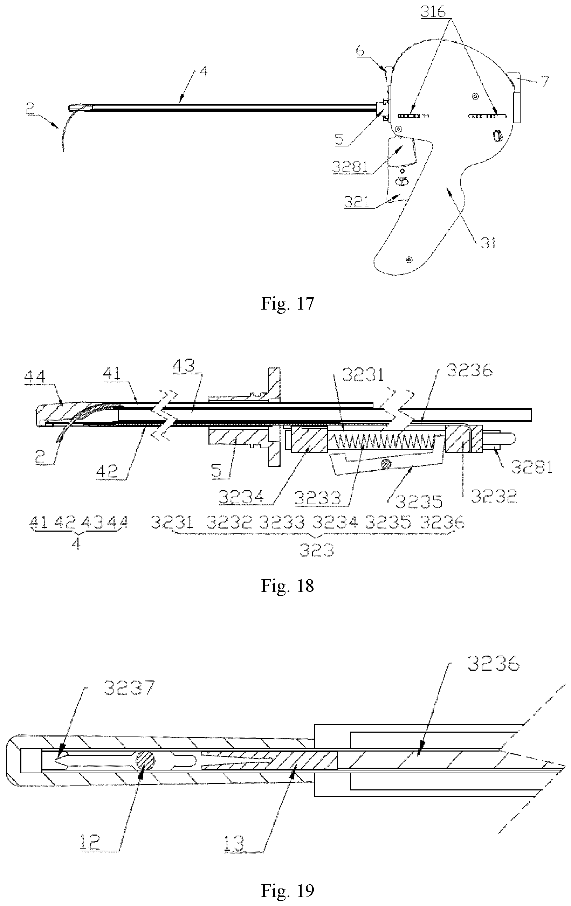

In the present embodiment, the first manipulation mechanism 327 serves as a safety mechanism which can move only when an external force is applied thereto. With continued reference to Figs. 9 and 10, in conjunction with Figs. 15 and 16, first of all, the first push member 3273 is pushed to one side to cause the first manipulation mechanism 327 to rotate so that the third limiting element 3271 no longer abuts against the manipulation member 3281. The manipulation member 3281 is then pressed to rotate, urging the fourth limiting element 3282 to abut against the first limiting element 3284 and thereby further causing the first limiting element 3284 to rotate away from the release member 325. After that, the release member 325 can be driven to rotate (for example, clockwise) over a predetermined angle to release the puncture needle (as shown in Fig. 17). Upon the release member 325 rotating to a limit position, the first abutment feature 3251 in the release member 325 hits and deforms the first limiting post 3241, allowing the limited withdrawal lock 321 to move in the third direction. Meanwhile, the third abutment feature 3252 comes into contact with the second abutment feature 3261. At this point, when the withdrawal lock 321 is pressed to rotate clockwise, the fifth limiting element 3212 will move away from the cutting lock 322, thus releasing the locked cutting lock 322. At the same time, the withdrawal lock 321 drives the transmission member 326 to rotate counterclockwise (under the assumption that the release member 325 is rotated clockwise in order to release the puncture needle). As a result, the second abutment feature 3261 comes into abutment against the third abutment feature 3252 and pushes the release member 325 to rotate counterclockwise to withdraw the puncture needle. Finally, the second push member 3224 is pushed to cause the cutting lock 322 to move, thus releasing the unlocked cutting and pushing mechanism 323.

It should be understood that, in the present embodiment, the power for driving the rotation of the release member 325 as required by the release of the puncture needle may be provided by a motor, a spring mechanism, a magnetic mechanism or the like. The present embodiment is not limited to any means for providing the power, and this means is not shown in the figures. For one skilled in the art, regardless of what kind of the means that provides the driving power, how to make it operable with the release member would be commonly known in the art and, therefore, need not be described in further detail herein. Additionally, how to cause the transmission member 326 to rotate counterclockwise as a result of the clockwise rotation of the withdrawal lock 321 would be commonly known to those skilled in the art. Further, the first manipulation mechanism 327, the second manipulation mechanism 328, the withdrawal lock 321 and the cutting lock 322 may be caused to move by various means such as pressing, rotating, sliding, etc., and the present invention is not limited to any particular such means.

During the unlocking of the trigger 32, a sound may be produced upon the release member 325 hitting the first limiting post 3251, providing the operator with an indication that the release member 325 has reached its limit position. In addition, ratchet teeth 3264 may be provided on the transmission member 326, and a contact tab 313 may be provided on the housing 31 into contact with the ratchet teeth 3264. As such, when the transmission member 326 is rotated, sounds will be produced as a result of the ratchet teeth 3264 successively coming into contact with the contact tab 313. Thus, when the transmission member 326 reaches the limit position and stops rotating, the operator would readily recognize this because sounds are no longer heard.

In alternative embodiments, the second manipulation mechanism 328 may not include the first limiting element 3284. In such embodiments, the fourth limiting element 3282 of the manipulation member 3281 may be adapted to lock the release member 325 instead by directly abutting against the release member 325.

Additionally, as shown in Figs. 8, 18 and 19, the cutting and pushing mechanism 323 includes a slide channel 3231, a first push block 3232, a biased push element 3233, a second push block 3234, a second limiting element 3235, a push rod 3236 and a cutter 3237. The slide channel 3231 may be an elongate channel arranged in the internal cavity. The first push block 3232 and the second push block 3234 are both movably provided on the slide channel 3231. The biased push element 3233 is coupled to both the first push block 3232 and the second push block 3234. The second limiting element 3235 is rotatably disposed within the internal cavity between the first push block 3232 and the second push block 3234. One end of the push rod 3236 is coupled to the first push block 3232, and the other end thereof is received in the second lumen 42 of the delivery tube 4 in order to push the proximal anchoring element 13 also received in the second lumen 42. The cutter 3237 is disposed in the second lumen 42 on the side of the proximal anchoring element 13 away from the housing 31. Moreover, the cutter 3237 is coupled to the second push block 3234. In the present embodiment, the biased push element 3233 includes, but is not limited to, a spring.

In the initial configuration, the biased push element 3233 is stretched (from its original length). The first push block 3232 is coupled to, and thus locked by, the locking hook 3222 of the cutting lock 322. The second limiting element 3235 abuts against the second push block 3234 and thereby keeps it stationary. When the cutting lock 322 is unlocked, it can be rotated by an external force to disengage the first push block 3232 from the cutting lock 322. As a result, the biased push element 3233 is released and moves the first push block 3232 in the slide channel 3231 toward the second push block 3234 (i.e., moves the first push block 3232 to the left as in the orientation of Fig. 18) until the first push block 3232 hits the second limiting element 3235. The hit second limiting element 3235 moves away from the second push block 3234, allowing the second push block 3234 to move in the slide channel 3231 toward the first push block 3232 (i.e., to the right) under the action of the biased push element 3233. In this process, the push rod 3236 moves to the left together with the first push block 3232, thus pushing the proximal anchoring element 13 to move in the same direction. Meanwhile, the cutter 3237 moves to the right together with the second push block 3234 until the proximal anchoring element 13 is attached to the connecting element 12 of the implant 1. After that, the cutter 3237 can be manipulated to cut off an undesired portion of the connecting element 12 from the implant 1.

As can be seen from the above description, in the initial configuration of the delivery system according to the present embodiment, the first manipulation mechanism 327, the second manipulation mechanism 328, the withdrawal lock 321, the cutting lock 322 and the cutting and pushing mechanism 323 are interlocked. In order to release the implant, the first manipulation mechanism 327 is first manipulated to release the locking of the second manipulation mechanism 328. The unlocked second manipulation mechanism 328 can be triggered to release the puncture needle and simultaneously release the locking of the withdrawal lock 321. The unlocked withdrawal lock 321 can be triggered to withdraw the puncture needle and simultaneously release the locking of the cutting lock 322. The unlocked cutting lock 322 can be triggered to release the locking of the cutting and pushing mechanism 323, and the unlocked cutting and pushing mechanism 323 can perform a cutting action. In this way, the components in the operating system must be triggered in a predetermined order to accomplish the implant release task without, avoiding failed release due to a wrong triggering order.

Optionally, the withdrawal lock 321 may lock the second limiting element 3235 in the initial configuration, and the locking may be released by an external force exerted on the withdrawal lock 321. Specifically, the withdrawal lock 321 may lock the second limiting element 3235 in the initial configuration by abutting against it and thereby preventing its rotation. When the withdrawal lock 321 is moved by an external force, the withdrawal lock 321 may disengage from, and thus release the locking of, the second limiting element 3235.

Additionally, in order for the second manipulation mechanism 328 to be more easily pressed, anti-slip features may be patterned in a press area of the manipulation member 3281 in the second manipulation mechanism 328. Likewise, anti-slip features may also be patterned in a press area of the withdrawal lock 321.

In the above release process of the implant delivery system, the implant may be stuck, for example, in the course of unlocking the release member 325 by rotating the second manipulation mechanism 328, or in the course of deforming the limiting mechanism 324 as a result of the release member 325 hitting the limiting mechanism 324, or in the course of unlocking the cutting and pushing mechanism 323 by rotating the cutting lock 322 in order to allow the first push block 3232 to hit the second limiting element 3235. The release of the implant will fail in any of those stuck cases. In order to address this, in another embodiment of the present invention, the operating system further includes a manual manipulation mechanism, and the housing 31 is further provided therein with windows, from which the manual manipulation mechanism can be inserted into the internal cavity to manually trigger the various components to release the stuck implant.

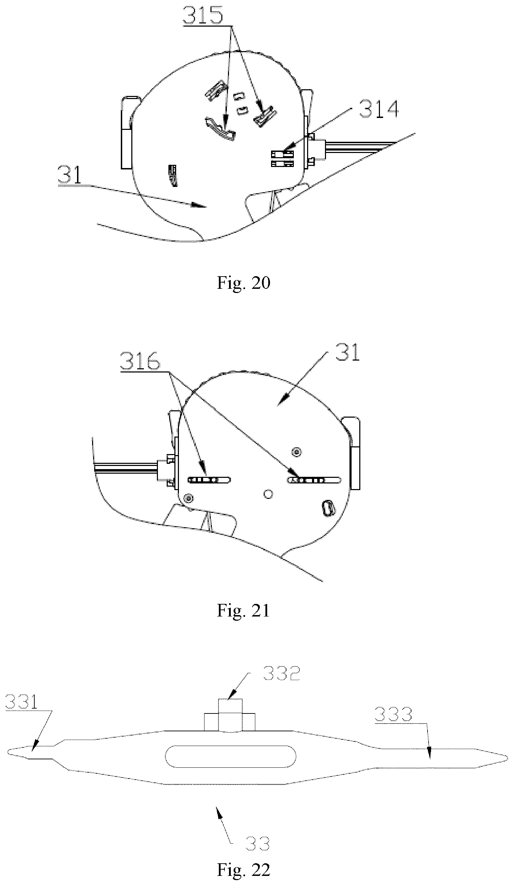

Referring to Figs. 20, 21 and 22, a first window 314 in communication with the internal cavity is provided in the housing 31. When the implant is stuck in the course of unlocking the release member 325, the manual manipulation mechanism 33 may be inserted into the internal cavity from the first window 314 to manipulate the second manipulation mechanism 328 to unlock the release member 325 from the second manipulation mechanism 328. Specifically, the first window 314 in the housing 31 is provided in positional correspondence with the manipulation member 3281 in order to allow the manual manipulation mechanism 33 to be inserted into the internal cavity from the first window 314 to exert a force on the manipulation member 3281 to cause it to rotate. The rotation of the manipulation member 3281 in turn causes the first limiting element 3284 to rotate away from the release member 325, thus releasing the locked release member 325. Alternatively, the first window 314 in the housing 31 may be provided in positional correspondence with the first limiting element 3284 in order to allow the manual manipulation mechanism 33 to be inserted into the internal cavity from the first window 314 to exert a force on the first limiting element 3284 to cause it to rotate away from the release member 325. In this embodiment, the first limiting element 3284 is preferably made of an elastic material which allows the first limiting element 3284 to deform under the action of the force exerted by the manual manipulation mechanism 33 to release the locked release member 325.

A second window 315 in communication with the internal cavity may be further provided in the housing 31. When the implant is stuck in the course of deforming the limiting mechanism 324 as a result of the release member 325 hitting the limiting mechanism 324, the manual manipulation mechanism 33 may be inserted into the internal cavity from the second window 315 to exert a force on the limiting mechanism 324 to cause its deformation. Specifically, the second window 315 in the housing 31 may be provided in positional correspondence with the first limiting post 3241 in order to allow the manual manipulation mechanism 33 to be inserted into the internal cavity from the second window 315 to exert a force on the first limiting post 3241. Preferably, the first limiting post 3241 is made of an elastic material.

A third window 316 in communication with the internal cavity may be further provided in the housing 31. When the implant is stuck in the course of the first push block 3232 hitting the second limiting element 3235, the manual manipulation mechanism 33 may be inserted into the internal cavity from the third window 316 to exert a force on the second limiting element 3235 to cause it to move away from the second push block 3234, thus releasing the locking of the second push block 3234.

In particular, the manual manipulation mechanism 33 may take a form depending on the shape and size of the various windows. Fig. 22 schematically illustrates a structure that the manual manipulation mechanism 33 may optionally employ. As shown in Fig. 22, the manual manipulation mechanism 33 includes a first release feature 331, a second release feature 332 and a third release feature 333. In this embodiment, the individual windows differ from one another in terms of size and shape. Accordingly, the first release feature 331, the second release feature 332 and the third release feature 333 are different from one another. In the illustrated case, the first release feature 331 is adapted for insertion into the internal cavity from the first window 314, the second release feature 332 is adapted for insertion into the internal cavity from the second window 315, and the third release feature 333 is adapted for insertion into the internal cavity from the third window 316. In alternative embodiments, the manual manipulation mechanism may have only one release feature which can be inserted into the internal cavity from any of the three windows.

As described above, in the implant delivery process implemented by the operating system in the delivery system according to the present embodiment, the interlocked mechanisms are sequentially triggered strictly according to a predetermined order to enable the release of the implant, ensuring safety and reliability of the delivery process. Moreover, the manual manipulation mechanism is provided to address the problem of the implant being stuck, additionally increasing the delivery system's effectiveness of use.

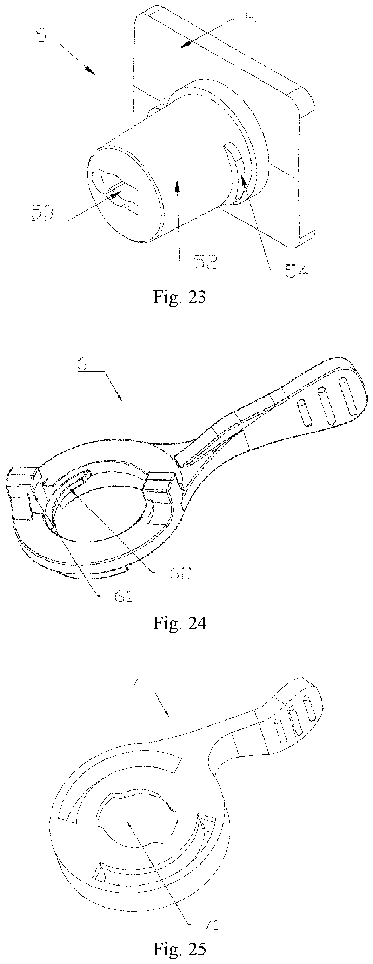

Further, as shown in Figs. 4 and 5, in practice, the delivery tube 4 is coupled to the housing 31 by means of a tube fitting 5. As shown in Fig. 23, the tube fitting 5 includes a flange 51 fixed to the housing 31. A coupling boss 52 projects from the flange 51, and a through bore 53 extends through both the coupling boss 52 and the flange 51 and communicates with the internal cavity, thus bringing the delivery tube 4 into communication with the internal cavity. Further, an arc-shaped fin 54 extends circumferentially on an outer wall of the coupling boss 52.

Furthermore, in order for accurate and reliable implant delivery to be achieved by the system, the implant delivery system may be further equipped with other devices such as an endoscope and sheath. Accordingly, as shown in Figs. 4 and 5, the implant delivery system further includes a sheath lock 6 and an endoscope lock 7.

As shown in Fig. 24, the sheath lock 6 includes a clamp lock 61 and an arc-shaped stop ring 62. The clamp lock 61 is adapted for attachment to the sheath, and the arc-shaped stop ring 62 is adapted to engage the arc-shaped fin 54. As shown in Fig. 25, the endoscope lock 7 is rotatably disposed on the housing 31 and includes a mount hole 71 adapted for retaining the endoscope. During the implant delivery process, the endoscope is inserted into the human body. Using the endoscope, it can be observed whether the puncture needle has carried the distal anchoring element to a predetermined site. Accordingly, as shown in Figs. 6 and 7, the delivery tube 4 further defines a third lumen 43 for receiving the endoscope therein. Walls of the first lumen 41, the second lumen 42 and the third lumen 43 are joined together so that the first lumen 41, the second lumen 42 and the third lumen 43 are arranged in a row along a direction perpendicular to an axis of the delivery tube 4. The delivery tube 4 further includes a tube tip 44 which is joined to each of the first lumen 41, the second lumen 42 and the third lumen 43. Moreover, the tube tip 44 is brought into communication with the first lumen 41 and thus defines therewith a puncture channel. The tube tip 44 is adapted to guide the puncture needle from linear movement to curved movement so that it travels from the first lumen 41 into the tube tip 44 and then penetrates into prostatic tissue from the tube tip 44. Further, in order to avoid sharp corners of the tube tip 44 from unnecessarily damaging human tissue, the tube tip 44 is wrapped with a protective film 45. It should be understood that one skilled in the art would well know how to construct the delivery tube 4, the tube fitting 5, the sheath lock 6 and the endoscope lock 7 and how to couple them to the housing 31.

On the basis of the above implant delivery system, examples of the present disclosure also provide an implant delivery method. Before the implant delivery begins, the operating system is in the initial configuration. Accordingly, the implant delivery method includes:
releasing the puncture needle;
releasing the locked withdrawal lock, releasing the locked cutting lock by applying an external force to the withdrawal lock, and withdrawing the puncture needle; and
releasing the cutting and pushing mechanism by applying an external force to the cutting lock so that the cutting and pushing mechanism is enabled to cut off the connecting element from the implant, thus completing the implant delivery process.

More specifically, the trigger includes a release member, a transmission member and a limiting mechanism. The puncture needle is coupled to the release member. The transmission member is coupled to the withdrawal lock, and the limiting mechanism is adapted to lock the withdrawal lock in the initial configuration.

An external force is exerted on the release member to drive it to rotate in a first direction, thereby releasing the puncture needle.

After the release member rotates over a predetermined angle, the release member completes the release of the puncture needle. At the same time, the release member hits and deforms the limiting mechanism, thereby releasing the locking of the withdrawal lock.

At the same time as an external force is applied to the withdrawal lock to unlock the cutting lock from the withdrawal lock, the withdrawal lock drives movement of the transmission member, which in turn causes the release member to rotate in a second direction to withdraw the puncture needle. The second direction is opposite to the first direction.

Optionally, the trigger further includes a release lock which is adapted to lock the release member in the initial configuration.

Prior to the application of an external force to the release member, the implant delivery method further includes unlocking the release member from the release lock by applying an external force to the release the lock.

Optionally, the release lock includes a first manipulation mechanism and a second manipulation mechanism. In the initial configuration, the first manipulation mechanism is adapted to lock the second manipulation mechanism, and the second manipulation mechanism is adapted to lock the release member.

Unlocking the release member from the release lock by applying an external force to the release lock include: unlocking the second manipulation mechanism from the first manipulation mechanism by applying an external force to the first manipulation mechanism; and then unlocking the release member from the second manipulation mechanism by applying an external force to the second manipulation mechanism.

Optionally, the second manipulation mechanism may be unlocked as a result of the first manipulation mechanism moving under the action of an external force; the release member may be unlocked as a result of the second manipulation mechanism moving under the action of an external force; the cutting lock may be unlocked as a result of the withdrawal lock moving under the action of an external force; and the cutting and pushing mechanism may be unlocked as a result of the cutting lock moving under the action of an external force. This interlocking design has the advantages that those components are unlocked and triggered according to a predetermined order during the implant delivery process of the delivery system, avoiding failed release due to a wrong unlocking order and improving the reliability and safety of the implant delivery system.

Optionally, if the implant is stuck in the course of unlocking the release member by moving the second manipulation mechanism, the manual manipulation mechanism is inserted into the internal cavity from the first window to manually urge the second manipulation mechanism to move.

Optionally, if the implant is stuck in the course of releasing the locked withdrawal lock as a result of the release member hitting the limiting mechanism, the manual manipulation mechanism is inserted into the internal cavity from the second window to manually urge the limiting mechanism to deform to unlock the withdrawal lock.

Optionally, if the implant is stuck in the course of unlocking the cutting and pushing mechanism by causing the cutting lock to move, the manual manipulation mechanism is inserted into the internal cavity from the third window to manually unlock the cutting and pushing mechanism by applying a force thereto.

The interlocked mechanisms in the delivery system are sequentially triggered strictly according to a predetermined order to enable the release of the implant, ensuring safety and reliability of the delivery process. Moreover, the manual manipulation mechanism is provided to address the problem of the implant being stuck, additionally increasing the delivery system's effectiveness of use.

Although the present invention has been disclosed hereinabove, it is not limited to the above disclosure. The invention is solely defined by the appended claims.

## Claims

1. An operating system (3) for use in an implant delivery system, the operating system (3) comprising a housing (31) and a trigger (32), the housing (31) defining an internal cavity in which part of the trigger (32) is movably disposed, the trigger (32) comprising a withdrawal lock (321), a cutting lock (322) and a cutting and pushing mechanism (323), the cutting and pushing mechanism (323) configured to cut off a connecting element (12) from an implant (1) delivered by the implant delivery system,
the operating system (3) having an initial configuration,
the trigger (32) configured so that, in the initial configuration, the withdrawal lock (321) is locked and locks the cutting lock (322), the cutting lock (322) locks the cutting and pushing mechanism (323), and that when the withdrawal lock (321) is unlocked, the withdrawal lock (321) releases the locked cutting lock under the action of an external force, followed by the cutting lock (322) releasing the locked cutting and pushing mechanism (323) under the action of an external force, wherein:
the trigger (32) further comprises a limiting mechanism (324), a release member (325) and a transmission member (326), the release member (325) is rotatably disposed in the internal cavity, the transmission member (326) is movably disposed in the internal cavity and is connected to the withdrawal lock (321),
**characterised in that**:
the limiting mechanism (324) is configured to lock the withdrawal lock (321) in the initial configuration, the release member (325) is configured to, when rotated in a first direction over a predetermined angle under the action of an external force, urge the limiting mechanism (324) to deform, thereby releasing the locked withdrawal lock, followed by the withdrawal lock (321) releasing the locked cutting lock under the action of an external force and simultaneously driving movement of the transmission member (326), the transmission member (326) further drives the release member (325) to rotate in a second direction opposite to the first direction.

2. The operating system (3) according to claim 1, wherein the trigger (32) further comprises a release lock configured to lock the release member (325) in the initial configuration.

3. The operating system (3) according to claim 2, wherein the release lock comprises a first manipulation mechanism (327) and a second manipulation mechanism (328), the first manipulation mechanism (327) configured to lock the second manipulation mechanism (328), the second manipulation mechanism (328) configured to lock the release member (325), in the initial configuration.

4. The operating system (3) according to claim 3, wherein the housing (31) is provided therein with a first window (314) in communication with the internal cavity, wherein the operating system (3) further comprises a manual manipulation mechanism (33), and
wherein the trigger (32) is further configured so that the second manipulation mechanism (328) releases the locked release member (325) under the action of a force exerted on the second manipulation mechanism (328) by the manual manipulation mechanism (33) inserted into the internal cavity from the first window (314).

5. The operating system (3) according to claim 4, wherein the second manipulation mechanism (328) comprises a manipulation member (3281) and a first limiting element (3284), the manipulation member (3281) disposed partially outside the housing (31) and partially in the internal cavity, the first limiting element (3284) disposed in the internal cavity, and wherein the second manipulation mechanism (328) is configured so that, in the initial configuration, the manipulation member (3281) abuts against the first manipulation mechanism (327), with the first limiting element (3284) abutting against both the manipulation member (3281) and the release member (325), thereby locking the release member (325), and that under the action of an external force, the manipulation member (3281) drives the first limiting element (3284) to move away from the release member (325), thereby releasing the locked release member;
optionally wherein:
(i) the first window (314) in the housing (31) is arranged in positional correspondence with the manipulation member (3281), and wherein the second manipulation mechanism (328) is further configured so that the manipulation member (3281) is able to drive the first limiting element (3284) to move away from the release member (325) and thus release the locked release member under the action of a force exerted on the manipulation member (3281) by the manual manipulation mechanism (33) inserted into the internal cavity from the first window (314); or
(ii) the first window (314) in the housing (31) is arranged in positional correspondence with the first limiting element (3284), and wherein the second manipulation mechanism (328) is further configured so that the first limiting element (3284) is able to release the locked release member under the action of a force exerted on the first limiting element (3284) by the manual manipulation mechanism (33) inserted into the internal cavity from the first window (314).

6. The operating system (3) according to claim 3, wherein the housing (31) is provided therein with a second window (315) in communication with the internal cavity, wherein the operating system (3) further comprises a manual manipulation mechanism (33), and wherein the trigger (32) is further configured so that the limiting mechanism (324) is able to deform to release the locked withdrawal lock under the action of a force exerted on the limiting mechanism (324) by the manual manipulation mechanism (33) inserted into the internal cavity from the second window (315);
optionally wherein
the limiting mechanism (324) comprises a first limiting post (3241) and a second limiting post (3242), and wherein the limiting mechanism (324) is configured so that the first limiting post (3241) limits movement of the withdrawal lock (321) in a third direction in the initial configuration and deforms to release the limited withdrawal lock under the action of an external force and that the second limiting post (3242) limits movement of the withdrawal lock (321) in a fourth direction opposite to the third direction;
further optionally wherein:
(i) the second window (315) in the housing (31) is arranged in positional correspondence with the first limiting post (3241), and wherein the first limiting post (3241) is able to deform under the action of a force exerted thereon by the manual manipulation mechanism (33) inserted into the internal cavity from the second window (315); or
(ii) the first limiting post (3241) is configured to abut against the transmission member (326) and thereby limit movement of the withdrawal lock (321) in the third direction.

7. The operating system (3) according to claim 3, wherein the cutting and pushing mechanism (323) comprises a slide channel (3231), a first push block (3232), a biased push element (3233), a second push block (3234) and a second limiting element (3235), the slide channel (3231) disposed in the internal cavity, the first and second push blocks movably provided on the slide channel (3231), the biased push element (3233) connected to both the first and second push blocks and configured to store potential energy, the second limiting element (3235) rotatably disposed in the internal cavity, and
wherein the cutting and pushing mechanism (323) is configured so that: in the initial configuration, the first push block (3232) is connected to the cutting lock (322) and is thus locked, the second limiting element (3235) is disposed between the first push block (3232) and the second push block (3234), and the second limiting element (3235) abuts against the second push block (3234) to lock the second push block (3234), and under the action of an external force exerted on the cutting lock (322), the first push block (3232) is disengaged from the cutting lock (322) and urged by the biased push element (3233) to move within the slide channel (3231) toward the second push block (3234) until the first push block (3232) comes into contact with and exerts a force on the second limiting element (3235), which causes the second limiting element (3235) to move away from the second push block (3234) and thus releases the locked second push block (3234), followed by the second push block (3234) being urged by the biased push element (3233) to move toward the first push block (3232);
optionally wherein:
(i) the housing (31) is provided therein with a third window (316) in communication with the internal cavity, wherein the operating system (3) further comprises a manual manipulation mechanism (33), and wherein the cutting and pushing mechanism (323) is further configured so that the second limiting element (3235) is able to move away from the second push block (3234) under the action of a force exerted on the second limiting element (3235) by the manual manipulation mechanism (33) inserted into the internal cavity from the third window (316); or
(ii) the withdrawal lock (321) is further configured to lock the second limiting element (3235) in the initial configuration and release the locked second limiting element (3235) under the action of an external force.

8. The operating system (3) according to claim 3, wherein the release member (325) defines a first abutment feature (3251) adapted to deform the limiting mechanism (324) by exerting a force thereon;
optionally wherein the transmission member (326) is rotatably disposed in the housing (31) so as to be coaxial with the release member (325) and defines a second abutment feature (3261) adapted to be brought into contact with the release member (325) and drive the release member (325) to rotate;
further optionally wherein the release member (325) further defines a third abutment feature (3252) adapted to be brought into contact with the second abutment feature (3261) of the transmission member (326) to enable the transmission member (326) to drive the release member (325) to rotate.

9. The operating system (3) according to claim 3, wherein:
(i) the transmission member (326) defines a protrusion (3262) adapted to be brought into contact with the limiting mechanism (324) and thus limit movement of the withdrawal lock (321) in a third direction; or
(ii) the transmission member (326) is provided with ratchet teeth (3264), and the housing (31) is provided with a contact tab (313), which is disposed in the internal cavity and brought into contact with the ratchet teeth (3264); or
(iii) the withdrawal lock (321) is rotatably disposed on the housing (31); and/or
(iv) the cutting lock (322) is rotatably disposed on the housing (31); and/or
(v) the first manipulation mechanism (327) is rotatably disposed on the withdrawal lock (321); and/or
(vi) the second manipulation mechanism (328) is rotatably disposed on the withdrawal lock (321);
optionally wherein the withdrawal lock (321) is provided with a push slot (3214) which is located outside the housing (31), and wherein the first manipulation mechanism (327) is provided with a push stud which is movably disposed in the push slot (3214).

10. The operating system (3) according to claim 1, wherein the housing (31) comprises a first housing and a second housing, which are joined together to delimit the internal cavity between the first housing and the second housing.

11. An implant delivery system for delivering an implant (1), the implant (1) provided with a connecting element (12), the implant delivery system comprising a puncture needle (2), a delivery tube (4) and the operating system (3) according to any one of claims 1 to 10,
the delivery tube (4) defining a first lumen (41) and a second lumen (42), the implant (1) having a first portion received in the puncture needle (2), the puncture needle (2) movably disposed in the first lumen, the implant (1) having a second portion disposed in the second lumen (42), part of the cutting and pushing mechanism (323) of the operating system (3) movably disposed in the second lumen (42), the cutting and pushing mechanism (323) configured to cut off the connecting element (12) from the implant (1).

## Patentansprüche

1. Operationssystem (3) zur Verwendung in einem Implantatabgabesystem, wobei das Operationssystem (3) ein Gehäuse (31) und einen Auslöser (32) umfasst, wobei das Gehäuse (31) einen inneren Hohlraum definiert, in dem ein Teil des Auslösers (32) beweglich angeordnet ist, wobei der Auslöser (32) eine Rückzugssperre (321), eine Schneidsperre (322) und einen Schneid- und Schiebemechanismus (323) umfasst, wobei der Schneid- und Schiebemechanismus (323) so konfiguriert ist, dass er ein Verbindungselement (12) von einem durch das Implantatabgabesystem abgegebenen Implantat (1) abschneidet,
wobei das Operationssystem (3) eine Anfangskonfiguration aufweist,
wobei der Auslöser (32) so konfiguriert ist, dass die Rückzugssperre (321) in der Anfangskonfiguration verriegelt ist und die Schneidsperre (322) verriegelt, die Schneidsperre (322) den Schneid- und Schiebemechanismus (323) verriegelt, und dass, wenn die Rückzugssperre (321) entriegelt wird, die Rückzugssperre (321) die verriegelte Schneidsperre unter der Wirkung einer äußeren Kraft freigibt, gefolgt davon, dass die Schneidsperre (322) den verriegelten Schneid- und Schiebemechanismus (323) unter der Wirkung einer äußeren Kraft freigibt, wobei:
der Auslöser (32) ferner einen Begrenzungsmechanismus (324), ein Freigabeglied (325) und ein Übertragungsglied (326) umfasst, das Freigabeglied (325) drehbar in dem inneren Hohlraum angeordnet ist, das Übertragungsglied (326) beweglich in dem inneren Hohlraum angeordnet ist und mit der Rückzugssperre (321) verbunden ist,
**dadurch gekennzeichnet, dass**:
der Begrenzungsmechanismus (324) so konfiguriert ist, dass er die Rückzugssperre (321) in der Anfangskonfiguration verriegelt, das Freigabeglied (325) so konfiguriert ist, dass es, wenn es in einer ersten Richtung über einen vorbestimmten Winkel unter der Wirkung einer äußeren Kraft gedreht wird, den Begrenzungsmechanismus (324) dazu drängt, sich zu verformen, wodurch die verriegelte Rückzugssperre freigegeben wird, gefolgt davon, dass die Rückzugssperre (321) die verriegelte Schneidsperre unter der Wirkung einer äußeren Kraft freigibt und gleichzeitig eine Bewegung des Übertragungsglieds (326) antreibt, das Übertragungsglied (326) ferner das Freigabeglied (325) antreibt, um sich in einer zur ersten Richtung entgegengesetzten zweiten Richtung zu drehen.

2. Operationssystem (3) nach Anspruch 1, wobei der Auslöser (32) ferner eine Freigabesperre umfasst, die so konfiguriert ist, dass sie das Freigabeglied (325) in der Anfangskonfiguration verriegelt.

3. Operationssystem (3) nach Anspruch 2, wobei die Freigabesperre einen ersten Manipulationsmechanismus (327) und einen zweiten Manipulationsmechanismus (328) umfasst, wobei der erste Manipulationsmechanismus (327) so konfiguriert ist, dass er den zweiten Manipulationsmechanismus (328) verriegelt, wobei der zweite Manipulationsmechanismus (328) so konfiguriert ist, dass er das Freigabeglied (325) in der Anfangskonfiguration verriegelt.

4. Operationssystem (3) nach Anspruch 3, wobei das Gehäuse (31) darin mit einem ersten Fenster (314) versehen ist, das mit dem inneren Hohlraum in Verbindung steht, wobei das Operationssystem (3) ferner einen manuellen Manipulationsmechanismus (33) umfasst, und
wobei der Auslöser (32) ferner so konfiguriert ist, dass der zweite Manipulationsmechanismus (328) das verriegelte Freigabeglied (325) unter der Wirkung einer Kraft freigibt, die auf den zweiten Manipulationsmechanismus (328) durch den manuellen Manipulationsmechanismus (33) ausgeübt wird, der vom ersten Fenster (314) in den inneren Hohlraum eingeführt wird.

5. Operationssystem (3) nach Anspruch 4, wobei der zweite Manipulationsmechanismus (328) ein Manipulationsglied (3281) und ein erstes Begrenzungselement (3284) umfasst, wobei das Manipulationsglied (3281) teilweise außerhalb des Gehäuses (31) und teilweise in dem inneren Hohlraum angeordnet ist, wobei das erste Begrenzungselement (3284) in dem inneren Hohlraum angeordnet ist, und wobei der zweite Manipulationsmechanismus (328) so konfiguriert ist, dass das Manipulationsglied (3281) in der Anfangskonfiguration an dem ersten Manipulationsmechanismus (327) anliegt, wobei das erste Begrenzungselement (3284) sowohl an dem Manipulationsglied (3281) als auch an dem Freigabeglied (325) anliegt, wodurch das Freigabeglied (325) verriegelt wird, und dass das Manipulationsglied (3281) unter der Wirkung einer äußeren Kraft das erste Begrenzungselement (3284) dazu antreibt, sich von dem Freigabeglied (325) wegzubewegen, wodurch das verriegelte Freigabeglied freigegeben wird;
wobei optional:
(i) das erste Fenster (314) in dem Gehäuse (31) in Positionsübereinstimmung mit dem Manipulationsglied (3281) angeordnet ist, und wobei der zweite Manipulationsmechanismus (328) ferner so konfiguriert ist, dass das Manipulationsglied (3281) dazu ausgelegt ist, das erste Begrenzungselement (3284) anzutreiben, um sich von dem Freigabeglied (325) wegzubewegen und somit das verriegelte Freigabeglied unter der Wirkung einer Kraft freizugeben, die auf das Manipulationsglied (3281) durch den manuellen Manipulationsmechanismus (33) ausgeübt wird, der vom ersten Fenster (314) in den inneren Hohlraum eingeführt wird; oder
(ii) das erste Fenster (314) in dem Gehäuse (31) in Positionsübereinstimmung mit dem ersten Begrenzungselement (3284) angeordnet ist, und wobei der zweite Manipulationsmechanismus (328) ferner so konfiguriert ist, dass das erste Begrenzungselement (3284) dazu ausgelegt ist, das verriegelte Freigabeglied unter der Wirkung einer Kraft freizugeben, die auf das erste Begrenzungselement (3284) durch den manuellen Manipulationsmechanismus (33) ausgeübt wird, der vom ersten Fenster (314) in den inneren Hohlraum eingeführt wird.

6. Operationssystem (3) nach Anspruch 3, wobei das Gehäuse (31) darin mit einem zweiten Fenster (315) versehen ist, das mit dem inneren Hohlraum in Verbindung steht, wobei das Operationssystem (3) ferner einen manuellen Manipulationsmechanismus (33) umfasst, und wobei der Auslöser (32) ferner so konfiguriert ist, dass der Begrenzungsmechanismus (324) dazu ausgelegt ist, sich zu verformen, um die verriegelte Rückzugssperre unter der Wirkung einer Kraft freizugeben, die auf den Begrenzungsmechanismus (324) durch den manuellen Manipulationsmechanismus (33) ausgeübt wird, der vom zweiten Fenster (315) in den inneren Hohlraum eingeführt wird;
wobei optional
der Begrenzungsmechanismus (324) eine erste Begrenzungssäule (3241) und eine zweite Begrenzungssäule (3242) umfasst, und wobei der Begrenzungsmechanismus (324) so konfiguriert ist, dass die erste Begrenzungssäule (3241) eine Bewegung der Rückzugssperre (321) in einer dritten Richtung in der Anfangskonfiguration begrenzt und sich verformt, um die begrenzte Rückzugssperre unter der Wirkung einer äußeren Kraft freizugeben, und dass die zweite Begrenzungssäule (3242) eine Bewegung der Rückzugssperre (321) in einer zur dritten Richtung entgegengesetzten vierten Richtung begrenzt;
wobei ferner optional:
(i) das zweite Fenster (315) in dem Gehäuse (31) in Positionsübereinstimmung mit der ersten Begrenzungssäule (3241) angeordnet ist, und wobei die zweite Begrenzungssäule (3241) dazu ausgelegt ist, sich unter der Wirkung einer Kraft zu verformen, die darauf durch den manuellen Manipulationsmechanismus (33) ausgeübt wird, der vom zweiten Fenster (315) in den inneren Hohlraum eingeführt wird; oder
(ii) die erste Begrenzungssäule (3241) so konfiguriert ist, dass sie an dem Übertragungsglied (326) anliegt und dadurch eine Bewegung der Rückzugssperre (321) in der dritten Richtung begrenzt.

7. Operationssystem (3) nach Anspruch 3, wobei der Schneid- und Schiebemechanismus (323) einen Gleitkanal (3231), einen ersten Schiebeblock (3232), ein vorgespanntes Schiebeelement (3233), einen zweiten Schiebeblock (3234) und ein zweites Begrenzungselement (3235) umfasst, wobei der Gleitkanal (3231) in dem inneren Hohlraum angeordnet ist, der erste und der zweite Schiebeblock beweglich an dem Gleitkanal (3231) vorgesehen sind, das vorgespannte Schiebeelement (3233) sowohl mit dem ersten als auch mit dem zweiten Schiebeblock verbunden und so konfiguriert ist, dass es potentielle Energie speichert, das zweite Begrenzungselement (3235) drehbar in dem inneren Hohlraum angeordnet ist, und
wobei der Schneid- und Schiebemechanismus (323) so konfiguriert ist, dass: in der Anfangskonfiguration der erste Schiebeblock (3232) mit der Schneidsperre (322) verbunden und somit verriegelt ist, das zweite Begrenzungselement (3235) zwischen dem ersten Schiebeblock (3232) und dem zweiten Schiebeblock (3234) angeordnet ist, und das zweite Begrenzungselement (3235) an dem zweiten Schiebeblock (3234) anliegt, um den zweiten Schiebeblock (3234) zu verriegeln, und unter der Wirkung einer äußeren Kraft, die auf die Schneidsperre (322) ausgeübt wird, der erste Schiebeblock (3232) von der Schneidsperre (322) gelöst wird und durch das vorgespannte Schiebeelement (3233) dazu gedrängt wird, sich innerhalb des Gleitkanals (3231) in Richtung des zweiten Schiebeblocks (3234) zu bewegen, bis der erste Schiebeblock (3232) in Kontakt mit dem zweiten Begrenzungselement (3235) kommt und eine Kraft darauf ausübt, was dazu führt, dass sich das zweite Begrenzungselement (3235) von dem zweiten Schiebeblock (3234) wegbewegt und somit den verriegelten zweiten Schiebeblock (3234) freigibt, gefolgt davon, dass der zweite Schiebeblock (3234) durch das vorgespannte Schiebeelement (3233) dazu gedrängt wird, sich in Richtung des ersten Schiebeblocks (3232) zu bewegen,
wobei optional:
(i) das Gehäuse (31) darin mit einem dritten Fenster (316) versehen ist, das mit dem inneren Hohlraum in Verbindung steht, wobei das Operationssystem (3) ferner einen manuellen Manipulationsmechanismus (33) umfasst, und wobei der Schneid- und Schiebemechanismus (323) ferner so konfiguriert ist, dass das zweite Begrenzungselement (3235) dazu ausgelegt ist, sich vom zweiten Schiebeblock (3234) unter der Wirkung einer Kraft wegzubewegen, die auf das zweite Begrenzungselement (3235) durch den manuellen Manipulationsmechanismus (33) ausgeübt wird, der von dem dritten Fenster (316) in den inneren Hohlraum eingeführt wird; oder
(ii) die Rückzugssperre (321) ferner so konfiguriert ist, dass sie das zweite Begrenzungselement (3235) in der Anfangskonfiguration verriegelt und das verriegelte zweite Begrenzungselement (3235) unter der Wirkung einer äußeren Kraft freigibt.

8. Operationssystem (3) nach Anspruch 3, wobei das Freigabeglied (325) ein erstes Auflageelement (3251) definiert, das dazu geeignet ist, den Begrenzungsmechanismus (324) durch Ausüben einer Kraft darauf zu verformen;
wobei optional das Übertragungsglied (326) drehbar in dem Gehäuse (31) angeordnet ist, so dass es koaxial mit dem Freigabeglied (325) ist, und ein zweites Auflageelement (3261) definiert, das dazu geeignet ist, in Kontakt mit dem Freigabeglied (325) gebracht zu werden und das Freigabeglied (325) zum Drehen anzutreiben;
wobei ferner optional das Freigabeglied (325) ferner ein drittes Auflageelement (3252) definiert, das dazu geeignet ist, mit dem zweiten Auflageelement (3261) des Übertragungsglieds (326) in Kontakt gebracht zu werden, um das Übertragungsglied (326) dazu zu befähigen, das Freigabeglied (325) zum Drehen anzutreiben.

9. Operationssystem (3) nach Anspruch 3, wobei:
(i) das Übertragungsglied (326) einen Vorsprung (3262) definiert, der dazu geeignet ist, mit dem Begrenzungsmechanismus (324) in Kontakt gebracht zu werden und somit eine Bewegung der Rückzugssperre (321) in einer dritten Richtung zu begrenzen; oder
(ii) das Übertragungsglied (326) mit Sperrzähnen (3264) versehen ist und das Gehäuse (31) mit einer Kontaktzunge (313) versehen ist, die in dem inneren Hohlraum angeordnet ist und mit den Sperrzähnen (3264) in Kontakt gebracht wird; oder
(iii) die Rückzugssperre (321) drehbar am Gehäuse (31) angeordnet ist; und/oder
(iv) die Schneidsperre (322) drehbar am Gehäuse (31) angeordnet ist; und/oder
(v) der erste Manipulationsmechanismus (327) drehbar an der Rückzugssperre (321) angeordnet ist; und/oder
(vi) der zweite Manipulationsmechanismus (328) drehbar an der Rückzugssperre (321) angeordnet ist;
wobei optional die Rückzugssperre (321) mit einem Schiebeschlitz (3214) versehen ist, der sich außerhalb des Gehäuses (31) befindet, und wobei der erste Manipulationsmechanismus (327) mit einem Schiebebolzen versehen ist, der beweglich in dem Schiebeschlitz (3214) angeordnet ist.

10. Operationssystem (3) nach Anspruch 1, wobei das Gehäuse (31) ein erstes Gehäuse und ein zweites Gehäuse umfasst, die zusammengefügt sind, um den inneren Hohlraum zwischen dem ersten Gehäuse und dem zweiten Gehäuse zu begrenzen.

11. Implantatabgabesystem zum Abgeben eines Implantats (1), wobei das Implantat (1) mit einem Verbindungselement (12) versehen ist, wobei das Implantatabgabesystem eine Punktionsnadel (2), ein Abgaberohr (4) und das Operationssystem (3) nach einem der Ansprüche 1 bis 10 umfasst,
wobei das Abgaberohr (4) ein erstes Lumen (41) und ein zweites Lumen (42) definiert, wobei das Implantat (1) einen ersten Abschnitt aufweist, der in der Punktionsnadel (2) aufgenommen ist, wobei die Punktionsnadel (2) beweglich in dem ersten Lumen angeordnet ist, wobei das Implantat (1) einen zweiten Abschnitt aufweist, der in dem zweiten Lumen (42) angeordnet ist, wobei ein Teil des Schneid- und Schiebemechanismus (323) des Operationssystems (3) beweglich in dem zweiten Lumen (42) angeordnet ist, wobei der Schneid- und Schiebemechanismus (323) so konfiguriert ist, dass er das Verbindungselement (12) von dem Implantat (1) abschneidet.

## Revendications

1. Système d'exploitation (3) destiné à être utilisé dans un système de pose d'implant, le système d'exploitation (3) comprenant un boîtier (31) et une gâchette (32), le boîtier (31) définissant une cavité interne dans laquelle une partie de la gâchette (32) est disposée de façon mobile, la gâchette (32) comprenant une serrure de retrait (321), une serrure de coupe (322) et un mécanisme de coupe et de poussée (323), le mécanisme de coupe et de poussée (323) étant configuré pour couper un élément de raccordement (12) d'un implant (1) posé par le système de pose d'implant,
le système d'exploitation (3) ayant une configuration initiale,
la gâchette (32) étant configurée de sorte que, dans la configuration initiale, la serrure de retrait (321) est verrouillée et verrouille la serrure de coupe (322), la serrure de coupe (322) verrouille le mécanisme de coupe et de poussée (323), et que lorsque la serrure de retrait (321) est déverrouillée, la serrure de retrait (321) débloque la serrure de coupe verrouillée sous l'action d'une force externe, suivie par le déblocage, par la serrure de coupe (322), du mécanisme de coupe et de poussée verrouillé (323) sous l'action d'une force externe, dans lequel :
la gâchette (32) comprend, en outre, un mécanisme de limitation (324), un élément de déblocage (325) et un élément de transmission (326), l'élément de déblocage (325) est disposé de façon rotative dans la cavité interne, l'élément de transmission (326) est disposé de façon mobile dans la cavité interne et est raccordé à la serrure de retrait (321),
**caractérisé en ce que** :
le mécanisme de limitation (324) est configuré pour verrouiller la serrure de retrait (321) dans la configuration initiale, l'élément de déblocage (325) est configuré pour, lorsqu'il est tourné dans un premier sens sur un angle prédéterminé sous l'action d'une force externe, inciter le mécanisme de limitation (324) à se déformer, débloquant de cette façon la serrure de retrait verrouillée, suivie par le déblocage, par la serrure de retrait (321), de la serrure de coupe verrouillée sous l'action d'une force externe et simultanément l'entraînement d'un mouvement de l'élément de transmission (326), l'élément de transmission (326) entraîne, en outre, l'élément de déblocage (325) à tourner dans un deuxième sens opposé au premier sens.

2. Système d'exploitation (3) selon la revendication 1, dans lequel la gâchette (32) comprend, en outre, une serrure de déblocage configurée pour verrouiller l'élément de déblocage (325) dans la configuration initiale.

3. Système d'exploitation (3) selon la revendication 2, dans lequel la serrure de déblocage comprend un premier mécanisme de manipulation (327) et un deuxième mécanisme de manipulation (328), le premier mécanisme de manipulation (327) étant configuré pour verrouiller le deuxième mécanisme de manipulation (328), le deuxième mécanisme de manipulation (328) étant configuré pour verrouiller l'élément de déblocage (325) dans la configuration initiale.

4. Système d'exploitation (3) selon la revendication 3, dans lequel le boîtier (31) est doté, à l'intérieur, d'une première fenêtre (314) en communication avec la cavité interne, dans lequel le système d'exploitation (3) comprend, en outre, un mécanisme de manipulation manuel (33), et
dans lequel la gâchette (32) est configurée, en outre, de sorte que le deuxième mécanisme de manipulation (328) débloque l'élément de déblocage verrouillé (325) sous l'action d'une force exercée sur le deuxième mécanisme de manipulation (328) par le mécanisme de manipulation manuel (33) inséré dans la cavité interne à partir de la première fenêtre (314).

5. Système d'exploitation (3) selon la revendication 4, dans lequel le deuxième mécanisme de manipulation (328) comprend un élément de manipulation (3281) et un premier élément de limitation (3284), l'élément de manipulation (3281) étant disposé partiellement à l'extérieur du boîtier (31) et partiellement dans la cavité interne, le premier élément de limitation (3284) étant disposé dans la cavité interne, et dans lequel le deuxième mécanisme de manipulation (328) est configuré de sorte que, dans la configuration initiale, l'élément de manipulation (3281) bute contre le premier mécanisme de manipulation (327), le premier élément de limitation (3284) butant à la fois contre l'élément de manipulation (3281) et l'élément de déblocage (325), verrouillant de cette façon l'élément de déblocage (325), et que sous l'action d'une force externe, l'élément de manipulation (3281) entraîne le déplacement du le premier élément de limitation (3284) à distance de l'élément de déblocage (325), débloquant de cette façon l'élément de déblocage verrouillé ;
dans lequel, facultativement :
(i) la première fenêtre (314) dans le boîtier (31) est agencée en correspondance de position avec l'élément de manipulation (3281), et dans lequel le deuxième mécanisme de manipulation (328) est configuré, en outre, de sorte que l'élément de manipulation (3281) est capable d'entraîner le premier élément de limitation (3284) à se déplacer à distance du premier élément de déblocage (325) et ainsi à débloquer l'élément de déblocage verrouillé sous l'action d'une force exercée sur l'élément de manipulation (3281) par le mécanisme de manipulation manuel (33) inséré dans la cavité interne à partir de la première fenêtre (314) ; ou
(ii) la première fenêtre (314) dans le boîtier (31) est agencée en correspondance de position avec le premier élément de limitation (3284), et dans lequel le deuxième mécanisme de manipulation (328) est configuré, en outre, de sorte que le premier élément de limitation (3284) est capable de débloquer l'élément de déblocage verrouillé sous l'action d'une force exercée sur le premier élément de limitation (3284) par le mécanisme de manipulation manuel (33) inséré dans la cavité interne à partir de la première fenêtre (314).

6. Système d'exploitation (3) selon la revendication 3, dans lequel le boîtier (31) est prévu à l'intérieur avec une deuxième fenêtre (315) en communication avec la cavité interne, dans lequel le système d'exploitation (3) comprend, en outre, un mécanisme de manipulation manuel (33), et dans lequel la gâchette (32) est configurée, en outre, de sorte que le mécanisme de limitation (324) est capable de se déformer pour débloquer la serrure de retrait verrouillée sous l'action d'une force exercée sur le mécanisme de limitation (324) par le mécanisme de manipulation manuel (33) inséré dans la cavité interne à partir de la deuxième fenêtre (315) ;
dans lequel, facultativement :
le mécanisme de limitation (324) comprend un premier montant de limitation (3241) et un deuxième montant de limitation (3242), et dans lequel le mécanisme de limitation (324) est configuré de sorte que le premier montant de limitation (3241) limite le mouvement de la serrure de retrait (321) dans un troisième sens dans la configuration initiale et se déforme pour débloquer la serrure de retrait limitée sous l'action d'une force externe et de sorte que le deuxième montant de limitation (3242) limite le mouvement de la serrure de retrait (321) dans un quatrième sens opposé au troisième sens ;
dans lequel, en outre, facultativement :
(i) la deuxième fenêtre (315) dans le boîtier (31) est agencée en correspondance positionnelle avec le premier montant de limitation (3241), et dans lequel le premier montant de limitation (3241) est capable de se déformer sous l'action d'une force exercée dessus par le mécanisme de manipulation manuel (33) inséré dans la cavité interne à partir de la deuxième fenêtre (315) ; ou
(ii) le premier montant de limitation (3241) est configuré pour buter contre l'élément de transmission (326) et limiter de cette façon le mouvement de la serrure de retrait (321) dans le troisième sens.

7. Système d'exploitation (3) selon la revendication 3, dans lequel le mécanisme de coupe et de poussée (323) comprend un canal de glissement (3231), un premier bloc de poussée (3232), un élément de poussée sollicité (3233), un deuxième bloc de poussée (3234) et un deuxième élément de limitation (3235), le canal de glissement (3231) étant disposé dans la cavité interne, les premier et deuxième blocs de poussée étant prévus de façon mobile sur le canal de glissement (3231), l'élément de poussée sollicité (3233) étant raccordé à la fois au premier et au deuxième blocs de poussée et configuré pour stocker l'énergie potentielle, le deuxième élément de limitation (3235) étant disposé de façon rotative dans la cavité interne, et
dans lequel le mécanisme de coupe et de poussée (323) est configuré de sorte que : dans la configuration initiale, le premier bloc de poussée (3232) est raccordé à la serrure de coupe (322) et est ainsi verrouillé, le deuxième élément de limitation (3235) est disposé entre le premier bloc de poussée (3232) et le deuxième bloc de poussée (3234), et le deuxième élément de limitation (3235) bute contre le deuxième bloc de poussée (3234) pour verrouiller le deuxième bloc de poussée (3234), et sous l'action d'une force externe exercée sur la serrure de coupe (322), le premier bloc de poussée (3232) est dégagé de la serrure de coupe (322) et incité, par l'élément de poussée sollicité (3233), à se déplacer à l'intérieur du canal de glissement (3231) vers le deuxième bloc de poussée (3234) jusqu'à ce que le premier bloc de poussée (3232) vienne en contact avec et exerce une force sur le deuxième élément de limitation (3235), ce qui provoque le déplacement du deuxième élément de limitation (3235) à distance du deuxième bloc de poussée (3234) et débloque ainsi le deuxième bloc de poussée verrouillé (3234), suivi par l'incitation du deuxième bloc de poussée (3234), par l'élément de poussée sollicité (3233), à se déplacer vers le premier bloc de poussée (3232) ;
facultativement, dans lequel :
(i) le boîtier (31) est doté à l'intérieur d'une troisième fenêtre (316) en communication avec la cavité interne, dans lequel le système d'exploitation (3) comprend, en outre, un mécanisme de manipulation manuel (33), et dans lequel le mécanisme de coupe et de poussée (323) est configuré, en outre, de sorte que le deuxième élément de limitation (3235) est capable de se déplacer à distance du deuxième bloc de poussée (3234) sous l'action d'une force exercée sur le deuxième élément de limitation (3235) par le mécanisme de manipulation manuel (33) inséré dans la cavité interne à partir de la troisième fenêtre (316) ; ou
(ii) la serrure de retrait (321) est, en outre, configurée pour verrouiller le deuxième élément de limitation (3235) dans la configure initiale et débloquer le deuxième élément de limitation verrouillé (3235) sous l'action d'une force externe.

8. Système d'exploitation (3) selon la revendication 3, dans lequel l'élément de déblocage (325) définit un premier élément de butée (3251) adapté pour déformer le mécanisme de limitation (324) en exerçant une force dessus ;
facultativement, dans lequel l'élément de transmission (326) est disposé de façon rotative dans le boîtier (31) de manière à être coaxial à l'élément de déblocage (325) et définit un deuxième élément de butée (3261) adapté pour être mis en contact avec l'élément de déblocage (325) et entraîner l'élément de déblocage (325) à tourner ;
en outre, facultativement, dans lequel l'élément de déblocage (325) définit, en outre, un troisième élément de butée (3252) adapté pour être mis en contact avec le deuxième élément de butée (3261) de l'élément de transmission (326) pour permettre à l'élément de transmission (326) d'entraîner l'élément de déblocage (325) à tourner.

9. Système d'exploitation (3) selon la revendication 3, dans lequel :
(i) l'élément de transmission (326) définit une saillie (3262) adaptée pour être mise en contact avec le mécanisme de limitation (324) et limiter ainsi le mouvement de la serrure de retrait (321) dans un troisième sens ; ou
(ii) l'élément de transmission (326) est doté de dents d'arrêt (3264), et le boîtier (31) est doté d'une patte de contact (313) qui est disposée dans la cavité interne et mise en contact avec les dents d'arrêt (3264) ; ou
(iii) la serrure de retrait (321) est disposée de façon rotative sur le boîtier (31) ; et/ou
(iv) la serrure de coupe (322) est disposée de façon rotative sur le boîtier (31) ; et/ou
(v) le premier mécanisme de manipulation (327) est disposé de façon rotative sur la serrure de retrait (321) ; et/ou
(vi) le deuxième mécanisme de manipulation (328) est disposé de façon rotative sur la serrure de retrait (321) ;
facultativement, dans lequel la serrure de retrait (321) est dotée d'une fente de poussée (3214) qui est située à l'extérieur du boîtier (31), et dans lequel le premier mécanisme de manipulation (327) est doté d'un goujon de poussée qui est disposé de façon mobile dans la fente de poussée (3214).

10. Système d'exploitation (3) selon la revendication 1, dans lequel le boîtier (31) comprend un premier boîtier et un deuxième boîtier qui sont réunis ensemble pour délimiter la cavité interne entre le premier boîtier et le deuxième boîtier.

11. Système de pose d'implant permettant de poser un implant (1), l'implant (1) étant doté d'un élément de raccordement (12), le système de pose d'implant comprenant une aiguille de ponction (2), un tube de pose (4) et le système d'exploitation (3) selon l'une quelconque des revendications 1 à 10,
le tube de pose (4) définissant une première lumière (41) et une deuxième lumière (42), l'implant (1) ayant une première portion reçue dans l'aiguille de ponction (2), l'aiguille de ponction (2) étant disposée de façon mobile dans la première lumière, l'implant (1) ayant une deuxième portion disposée dans la deuxième lumière (42), une partie du mécanisme de coupe et de poussée (323) du système d'exploitation (3) étant disposée de façon mobile dans la deuxième lumière (42), le mécanisme de coupe et de poussée (323) étant configuré pour couper l'élément de raccordement (12) de l'implant (1).
